# EUROPEAN PATENT SPECIFICATION

(11) **EP 1 581 340 B1**
(45) Date of publication and mention of the grant of the patent: **23.02.2011**
(21) Application number: 03700020.5
(22) Date of filing: 03.01.2003
(51) Int. Cl.: B01J 27/198, B01J 37/03, B01J 37/06, C07C 51/215

(54) **PROCESS FOR PREPARING VANADYL PYROPHOSPHATE CATALYST**
VERFAHREN ZUR HERSTELLUNG VON VANADYLPYROPHOSPHAT-KATALYSATOREN
PROCEDE DE PREPARATION DE CATALYSEUR DE PYROPHOSPHATE DE VANADYLE

(43) Date of publication of application: 05.10.2005
(73) Proprietor: Council of Scientific and Industrial Research, New Delhi 100 001 (IN)
(72) Inventor: DATTA, Arunabha, Dehradun 248 005 Uttaranchal (IN); DASGUPTA, Soumen, Dehradun 248 005 Uttaranchal (IN); AGARWAL, Monika c/o Prof. Ramanan, Haus Khas, New Dehli 110 017 (IN)
(74) Representative: Mattsson, Niklas
(86) International application number: PCT/IB2003/000016
(87) International publication number: WO 2004/060558

(56) References cited:
- HOROWITZ H.S. ET AL: "V-P-O CATALYSTS FOR OXIDATION OF BUTANE TO MALEIC ANHYDRIDE" APPLIED CATALYSIS, AMSTERDAM, NL, vol. 38, 1988, pages 193-210, XP009011793 ISSN: 0166-9834
- MIZUNO N ET AL: "One-Pot Synthesis of VOHPO4*0,5H2O with High Growth of the (001) Plane: An Important Catalyst Precursor of (VO)2P2O7" CHEMISTRY OF MATERIALS, AMERICAN CHEMICAL SOCIETY, WASHINGTON, US, vol. 9, 1997, pages 2697-2698, XP002243361 ISSN: 0897-4756 cited in the application
- DATABASE WPI Section Ch, Week 199321 Derwent Publications Ltd., London, GB; Class A41, AN 1993-171967 XP002243362 & JP 05 103990 A (TOSOH CORP), 27 April 1993 (1993-04-27)
- HUTCHINGS G J ET AL: "Selective oxidation of n-butane to maleic anhydride with vanadium phosphorus catalysts prepared by comminution in the presence of dispersants" APPLIED CATALYSIS A: GENERAL, ELSEVIER SCIENCE, AMSTERDAM, NL, vol. 154, no. 1-2, 12 June 1997 (1997-06-12), pages 103-115, XP004338094 ISSN: 0926-860X

## Description

### Field of the invention

The present invention relates to a process for preparing vanadyl pyrophosphate catalyst. More particularly, the present invention relates to a process for the preparation of vanadyl pyrophosphate catalyst with improved structural characteristics for the selective oxidation of butane to maleic anhydride.

### Background of the invention

Layered vanadyl hydrogen phosphate hemihydrate, VOHPO₄.0.5H₂O has tremendous technological importance as the precursor to the vanadyl pyrophosphate, (VO)₂P₂O₇ which is used as the catalyst for the commercially established selective oxidation of butane to maleic anhydride [**Ref**. G. Centi, F. Trifiro, J. R. Ebner, V. M. Franchetti, Chem.Rev. ,1988, vol. 58, p 55].

Maleic anhydride is a valuable intermediate for the production of some commercially important fine chemicals and polymers. The largest use of maleic anhydride occurs in the production of unsaturated polyesters. In the fine chemicals industry, maleic anhydride is used as a raw material for the production of succinic anhydride, γ-butyrolactone, 1,4-butanediol, tetrahydrofuran, fumaric acid, malic acid and D-L tartaric acid.

Vanadyl pyrophosphate (VO)₂P₂O₇ catalyzed process of maleic anhydride synthesis by selective oxidation of butane replaced the earlier process of gas phase oxidation of benzene on a supported V-Mo-O catalyst due to economic and environmental reasons. Under typical industrial conditions 65-70% selectivity towards maleic anhydride is achieved for butane conversion of 70-85%. Carbondioxide and trace amounts of acetic acid are the only other byproducts.

Vanadyl pyrophosphate (VO)₂P₂O₇ is regarded to be the active phase of the VPO catalyst since it is the only bulk phase that exists in equilibrated VPO catalysts. The catalytic activity of (VO)₂P₂O₇ is known to be sensitive to its morphological characteristics and in particular the preferential exposure of the ***(100)*** crystallographic plane of the (VO)₂P₂O₇ crystallites is highly desirable since this plane has been established to be the most active and selective for the oxidation of butane to maleic anhydride **[Ref.** "Vanadyl Pyrophosphate Catalysts", ed. G. Centi, Catal. Today, 1993, vol. 16]. The morphology of the VO₂P₂O₇ phase is indirectly controlled through that of the VOHPO₄.0.5H₂O phase, since it undergoes a topotactic transformation at - 450°C to the (VO)₂P₂O₇ phase with retention of the microstructure and morphological characteristics of the precursor VOHPO₄.0.5H₂O phase **[Ref.** J. W. Johnson, D. C. Johnston. A. J. Jacobson, J. F. Brody, J. Am. Chem. Soc., 1984, vol. 106, p. 8123]. During this transformation the ***(001)*** plane of VOHPO₄.0.5H₂O is transformed to the active ***(100)*** plane of (VO)₂P₂O₇ with retention of the morphology/surface defect of the ***(001)*** plane of the hemihydrate phase.

In view of the well established role of the ***(100)*** plane of the (VO)₂P₂O₇ phase in catalyzing the selective oxidation of butane to maleic anhydride, the synthesis of phases with preferential exposure of this plane would be of great significance in increasing the activity of (VO)₂P₂O₇ catalyst. The intensity ratio of the interplaner *(100)* and in-plane *(021)* x-ray reflection of (VO)₂P₂O₇ i.e. *(I₁₀₀:I₀₂₁)* is used to determine the preferential exposure of the surface *(100)* planes proposed to contain the active and selective catalytic sites for selective oxidation of butane to maliec anhydride. The conventional methods of synthesis of (VO)₂P₂O₇ catalysts typically exibits low intensity ratios *I₁₀₀:I₀₂₁* ca. 0.4-2 indicating that the surface *(100)* planes are not preferentially exposed by these methods **[Ref.** V. V. Guliants, J. B. Benziger, S. Sundaresan, I. E. Wachs, J. M. Jehng, J. E. Roberts, Catal. Today, 1996, voL 28, p. 275].

There are very few reports in literature on the preferential exposure of catalytically important *(100)* crystallographic plane of (VO)₂P₂O₇ phase.

Reference is made to a procedure of one pot synthesis of VOHPO₄.0.5H₂O with high growth of the *(001)* plane and its subsequent transformation to a (VO)₂P₂O₇ phase with preferentially exposed *(100)* plane [N. Mizuno, H. Hatayama, M. Misono, Chem. Mater., 1997, vol. 9, no. 12, p. 2697]. The drawback of this procedure is the requirement of a costly surfactant and hydrothermal condition during the crystallization of the precursor VOHPO₄.0.5H₂O phase.

Reference is made to colloidal templated synthesis of (VO)₂P₂O₇ phase [M.A. Carreon, V.V.Guliants, Chem.Commun., 2001, p 1438] with *I₁₀₀:I₀₂₁* = 2.48 suggesting preferential exposure of surface *(100)* planes. However, this method comprises a complex procedure using mono dispersed polystyrene spheres as templating agents for formation of VO)₂P₂O₇ with selectively exposed catalytically important *(100)* plane.

### Objects of the invention

The main object of the invention is to provide a process for the preparation of vanadyl pyrophosphate catalysts with improved structural characteristics for the selective oxidation of butane to maleic anhydride.

Another object of the invention is to provide a simple method for the selective exposure of catalytically active *(100)* plane of the (VO)₂P₂O₇ catalyst used for selective oxidation of butane to maleic anhydride.

Yet another object of the invention is to provide a process for the preparation of vanadyl pyrophosphate catalyst which exhibits high selective exposure of the catalytically active *(100)* plane in the (VO)₂P₂O₇ catalyst in comparison to catalyst prepared by conventional methods.

It is another object of the invention to provide a process for the preparation of vanadyl pyrophosphate which does not require hydrothermal reaction conditions for the preparation of (VO)₂P₂O₇ catalyst with selective exposure of the catalytically important *(100)* plane.

It is further object of the invention to provide a process for the preparation or vanadyl pyrophosphate which does not require costly surfactant and mono dispersed colloidal templating agents during the preparation of (VO)₂P₂O₇ catalyst with selectively exposed *(100)* plane.

### Summary of the invention

Accordingly the present invention provides a process for the preparation of vanadyl pyrophosphate catalyst with improved structural characteristics and useful for the selective oxidation of butane to maleic anhydride, which comprises:
i) reducing V₂O₅ solid by an aqueous solution of NH₂OH.HCl in the presence of H₃PO₄ to obtain a blue solution w,
ii) evaporating the solution to obtain a pasty mass,
iii) aging the pasty mass to obtain a blue solid,
iv) washing the solid with boiling water to remove the water soluble phases,
v) drying the solid to form VOHPO₄.0.5H₂O phase,
vi) grinding the dry VOHPO₄.0.5H₂O phase to a fine powder,
vii) dispersing the VOHPO₄.0.5H₂O powder into a mixture of dimethyl formamide (DMF) and water (H₂O) and stirring the resulting slurry,
viii) recovering the dispersed VOHPO₄.0.5H₂O powder from the slurry and washing with hot water,
ix) drying the powder to obtain a VOHPO₄.0.5H₂O solid with selectively enhanced *(001)* plane,
x) calcining the VOHPO₄.0.5H₂O solid with selectively enhanced *(001)* plane to obtain the desired (VO)₂P₂O₇ phase with selectively exposed *(100)* plane.

In one embodiment of the invention, V₂O₅ solid is reduced in step (i) at a temperature in the range of 70-100°C while maintaining a P:V ratio in the reaction mixture in the range of 1:0.8 to 1:1.4.

In another embodiment of the invention, the pasty mass obtained in step (ii) is aged in step (iii) by heating to a temperature in the range of 100-150°C in air atmosphere for a period in the range of 12-36 hours.

In yet another embodiment of the invention, the washed solid obtained in step (iv) is dried in step (v) at a temperature in the range of 100-150°C for 6-16 hours in air atmosphere to form VOHPO₄.0.5H₂O phase.

In yet another embodiment of the invention, the dry VOHPO₄.0.5H₂O powder obtained in step (vi) is dispersed in step (vii) in a mixture of dimethyl formamide (DMF) and water (H₂O) wherein the DMF to H₂O ratio is in the range of 20:1-1:20 (v/v) and the resulting slurry is stirred for 1-6 hours at a temperature in the range of 50-100°C,

In another embodiment of the invention, the dispersed VOHPO₄.0.5H₂O powder is recovered from the slurry of step (vii) by filtration.

In yet another embodiment of the invention, the VOHPO₄.0.5H₂O solid with selectively enhanced *(001)* plane obtained at the end of step (ix) is calcined in step (x) at a temperature in the range of 400 -500°C under flowing nitrogen, at flow rate of 6-10 litre/hour for 1-6 hours to obtain desired (VO)₂P₂O₇ phase with selectively exposed *(100)* plane.

In another embodiment of the invention the ratio of DMF to H₂O used in the mixture of DMF and H₂O is preferably in the range of 2:1-1:2 (v/v).

In another embodiment of the invention, the temperature during stirring of VOHPO₄.0.5H₂O powder in DMF-H₂O mixture is preferably in the range of 75-90°C.

In another embodiment of the invention, the VOHPO₄.0.5H₂O solid obtained shows selective exposure of the *(001)* plane.

In another embodiment of the invention, selective exposure of the *(001)* plane in VOHPO₄.0.5H₂O can be varied by changing the duration of DMF-H₂O treatment.

In another embodiment of the invention, the temperature used in calcination of VOHPO₄.0.5H₂O solid is in the range of 450-470°C with a gradual increase of temperature at a rate of 1-2°C/ minute under flowing nitrogen.

In another embodiment of the invention, the (VO)₂P₂O₇ obtained shows enhanced exposure of the *(100)* plane and is catalytically active for the selective oxidation of butane to maleic anhydride.

### Brief description of the accompanying drawing

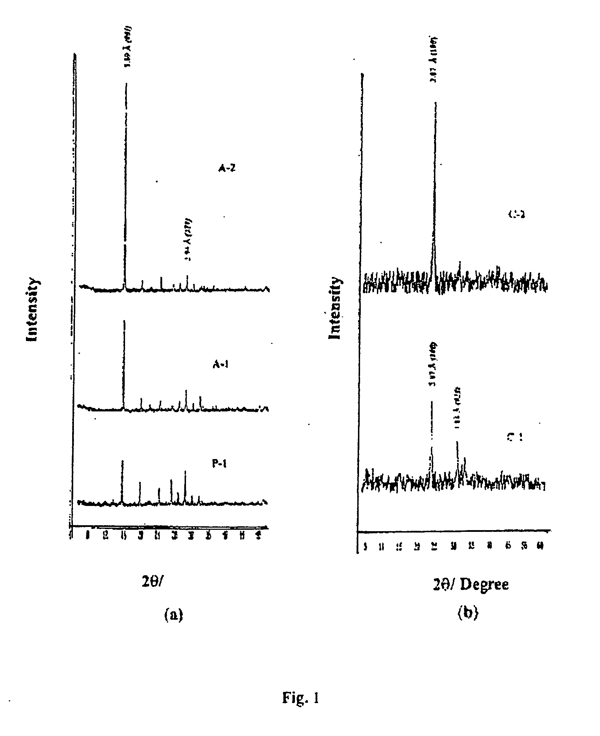

Figure 1a shows XRD pattern of the as synthesized VOHPO₄.0.5H₂O phase (sample code P-0) and the VOHPO₄.0.5H₂O phases obtained after stirring of the as synthesized VOHPO₄-0.5H₂O phase in DMF-H₂O mixture at 80°C for 2 hours (sample code P-2) and 4 hours (sample code P-4), indicating selective exposure of the *(001)* plane topotactically related to the catalytically important *(100)* plane of the (VO)₂P₂O₇ catalyst.

Figure 1b shows XRD pattern of (VO)₂P₂O₇ phase (sample code C-0) obtained by thermal topotactic transformation of the as synthesized precursor VOHPO₄.0.5H₂O (P-0) and the XRD of the (VO)₂P₂O₇ phase (sample code C-4) obtained by transformation of the precursor P-4 (defined in table 1) showing selective exposure of the *(100)* plane in the (VO)₂P₂O₇ phase transformed from DMF-H₂O treated VOHPO₄.0.5H₂O precursor.

### Detailed description of the invention

The present invention provides a process for the preparation of vanadyl pyrophosphate catalyst with improved structural characteristics and useful for the selective oxidation of butane to maleic anhydride. The process of the invention comprises reducing V₂O₅ solid by an aqueous solution of NH₂OH.HCl in the presence of H₃PO₄ to obtain a blue solution w, evaporating the solution to obtain a pasty mass, aging the pasty mass to obtain a blue solid, washing the solid with boiling water to remove the water soluble phases, drying the solid to form VOHPO₄.0.5H₂O phase, grinding the dry VOHPO₄.0.5H₂O phase to a fine powder, dispersing the VOHPO₄.0.5H₂O powder into a mixture of dimethyl formamide (DMF) and water (H₂O) and stirring the resulting slurry, recovering the dispersed VOHPO₄.0.5H₂O powder from the slurry and washing with hot water, drying the powder to obtain a VOHPO₄.0.5H₂O solid with selectively enhanced *(001)* plane, calcining the VOHPO₄.0.5H₂O solid with selectively enhanced *(001)* plane to obtain the desired (VO)₂P₂O₇ phase with selectively exposed *(100)* plane.

The V₂O₅ solid is reduced in step (i) preferably at a temperature in the range of 70-100°C while maintaining a P:V ratio in the reaction mixture in the range of 1:0.8 to 1: 1.4. The pasty mass obtained in step (ii) is aged in step (iii) by heating to a temperature in the range of 100-150°C in air atmosphere for a period in the range of 12-36 hours. The washed solid obtained in step (iv) is dried in step (v) preferably at a temperature in the range of 100-150°C for 6-16 hours in air atmosphere to form VOHPO₄.0.5H₂O phase. The dry VOHPO₄.0.5H₂O powder obtained in step (vi) is dispersed in step (vii) in a mixture of dimethyl formamide (DMF) and water (H₂O) with DMF to H₂O ratio in range of 20:1-1:20 (v/v). The resulting slurry is stirred for 1-6 hours at a temperature in the range of 50-100°C. Dispersed VOHPO₄.0.5H₂O powder is then recovered from slurry of step (vii) by filtration.

The VOHPO₄.0.5H₂O solid with selectively enhanced *(001)* plane obtained at the end of step (ix) is calcined in step (x) preferably at a temperature in the range of 400 -500°C under flowing nitrogen, at flow rate of 6-10 litre/hour for 1-6 hours to obtain desired (VO)₂P₂O₇ phase with selectively exposed *(100)* plane. The ratio of DMF to H₂O used in the mixture of DMF and H₂O is preferably in the range of 2:1-1:2 (v/v). The temperature during stirring of VOHPO₄.0.5H₂O powder in DMF-H₂O mixture is preferably in the range of 75-90°C. The VOHPO₄.0.5H₂O solid obtained shows selective exposure of the *(001)* plane. Selective exposure of the *(001)* plane in VOHPO₄.0.5H₂O can be varied by changing duration of DMF-H₂O treatment. The temperature used in calcination of VOHPO₄.0.5H₂O solid is in the range of 450-470°C with a gradual increase of temperature at the rate of 1-2°C/ minute under flowing nitrogen.

The (VO)₂P₂O₇ obtained shows enhanced exposure of the *(100)* plane and is catalytically active for the selective oxidation of butane to maleic anhydride. In the present invention infrared spectra (FTIR), thermo-gravimetric analysis (TGA) and elemental analysis showed no structural changes in VOHPO₄.0.5H₂O solid following its treatment in DMF-H₂O solvent mixture. X-ray diffraction analysis of the (VO)₂P₂O₇ phases, obtained by thermal transformation of the VOHPO₄.0.5H₂O phase with DMF-H₂O treatment, shows a 4 to 8 fold increase of the ratio of intensity of the most prominent *(100)* line to the next most prominent *(021)* line, i.e. *I₁₀₀*/*I₀₂₁* in comparison to the (VO)₂P₂O₇ phase obtained by thermal transformation of the VOHPO₄.0.5H₂O solid without any DMF-H₂O treatment.

The following representative example is given by way of illustration and therefore should not be construed to limit the scope of the present invention.

### Example

### Step 1:

3.475 grams (0.05 moles) of hydroxylamine hydrochloride (99.99 % purity, Sigma) was dissolved in laboratory-distilled water (150 ml). Into this solution, 4.545 grams (0.025 moles) vanadium pentoxide (99.99 % purity, Sigma) powder was dispersed followed by 5.76 grams (0.05 moles) of 85% phosphoric acid (Sigma) and the slurry magnetically stirred at 80°C for 2 hours. During this time the orange yellow slurry gradually turned into an ink blue solution. This solution was evaporated to a blue pasty mass and aged in an air oven at 120°C for 16 hours. The sky blue solid obtained was finely ground and the powder rinsed several times with boiled distilled water till no chloride ion was detected in the washing. The solid was dried in an air oven at 120°C for 16 hours. XRD, elemental analysis and FTIR characterization confirmed the formation of pure VOHPO₄.0.5H₂O phase.

### Step 2:

Two gm quantity of VOHPO₄.0.5H₂O prepared in step 1 was dispersed into a 30-ml. mixture of 1:1 volume/volume dimethyl formamide (99 % purity, Ranbaxy Chemicals) and laboratory-distilled water. The slurry was stirred at 80°C for 2 hours. The solid was isolated by filtration, washed thoroughly with warm water and dried in an air oven at 120°C for 6 hours. XRD showed selective enhancement of the intensity of the *(001)* reflection of VOHPO₄.0.5H₂O. Thus the ratio of the intensity due to the *(001)* and the next most intense *(220)* reflection in VOHPO₄.0.5H₂O increased from 1.4 to 10 on DMF-H₂O treatment for 2 hours indicating selectively enhanced exposure of the *(001)* plane of VOHPO₄.0.5H₂O.

### Step 3:

One-gram quantity of DMF-H₂O treated VOHPO₄.0.5H₂O solid with selectivity exposed *(001)* plane and the parent VOHPO₄.0.5H₂O solid without DMF-H₂O treatment was calcined at 450°C in a furnace for 2 hours. The calcination temperature was gradually attained at a linear rate of 2 °C/minute. Throughout the heating process dry nitrogen was continuously passed over the solids. XRD of the brown solids obtained shows the formation of pure (VO)₂P₂O₇ phase in both the cases. The intensity ratio of the *(100)* and the next intense *(021)* reflection i.e. I₁₀₀/I₀₂₁ increased from 2 in the (VO)₂P₂O₇ phase obtained by thermal transformation of the VOHPO₄.0.5H₂O phase without DMF-H₂O treatment to 10.8 in the (VO)₂P₂O₇ phase obtained by thermal transformation of the VOHPO₄.0.5H₂O phase, stirred in DMF-H₂O mixture for 2 hours, showing higher selective exposure of the *(100)* planes in the later phase.

**Table 1:**

| Intensity ratio of XRD lines due to *(001)* and *(220)* reflection i.e. I₀₀₁/I₂₂₀ of as synthesized VOHPO₄.0.5H₂O (sample code **P-0)** and VOHPO₄.0.5H₂O stirred in 1:1 V/V DMF-H₂O mixture at 80°C for different durations. | | |
|---|---|---|
| Sample Code | Duration of stirring (In hours) | I₀₀₁/I₂₂₀ |
| **P-0** | 0 | 1.4 |
| **P-1** | 1 | 8 |
| **P-2** | 2 | 10 |
| **P-4** | 4 | 14 |
| **P-6** | 6 | 14.5 |

**Table 2:**

| Intensity ratio of XRD lines due to *(100)* and *(021)* reflection i.e. I₁₀₀/I₀₂₁ of (VO)₂P₂O₇ phase transformed from as synthesized VOHPO₄.0.5H₂O (sample code C-0) and from VOHPO₄.0.5H₂O precursor stirred in 1:1 volume/volume DMF-H₂O mixture at 80 °C for different durations. | | |
|---|---|---|
| Sample Code | Duration of stirring of the precursor in DMF-H₂O mixture. (In hours) | I₁₀₀/I₀₂₁ |
| **C-0** | 0 | 2 |
| **C-1** | 1 | 8.5 |
| **C-2** | 2 | 10.8 |
| **C-4** | 4 | 12 |
| **C-6** | 6 | 12.4 |

Fig. 1a shows XRD pattern of the as synthesized VOHPO₄.0.5H₂O phase (sample code P-0) and the VOHPO₄.0.5H₂O phases obtained after stirring of the as synthesized VOHPO₄.0.5H₂O phase in DMF-H₂O mixture at 80 °C for 2 hours (sample code P-2) and 4 hours (sample code P-4), indicating selective exposure of the *(001)* plane topotactically related to the catalytically important *(100)* plane of the (VO)₂P₂O₇ catalyst.

Fig. 1b shows XRD pattern of (VO)₂P₂O₇ phase (sample code C-0) obtained by thermal topotactic transformation of the as synthesized precursor VOHPO₄.0.5H₂O (P-0) and the XRD of the (VO)₂P₂O₇ phase (sample code C-4) obtained by transformation of the precursor P-4 (defined in table 1) showing selective exposure of the *(100)* plane in the (VO)₂P₂O₇ phase transformed from DMF-H₂O treated VOHPO₄.0.5H₂O precursor.
The main advantages of the present invention are:
1. The process of the present invention provides a simple method for selective exposure of the catalytically active *(100)* plane of the (VO)₂P₂O₇ catalyst used for the selective oxidation of butane to maleic anhydride.
2. The present process demonstrate that by a simple post synthetic solvent treatment in DMF-H₂O or DMSO-H₂O mixture, the growth of the *(001)* plane of the VOHPO₄.0.5H₂O phase can be selectivity enhanced which subsequently can be translated by a thermal topotactic transformation to the selective growth of the catalytically active *(100)* plane of the (VO)₂P₂O₇ phase.
3. The conventional (VO)₂P₂O₇ catalyst typically exhibited low intensity ratio *I₁₀₀*/*I₀₂₁* ca. 0.4-2 whereas the process described achieves *I₁₀₀*/*I₀₂₁* ratio ca. 8-12 indicating higher selective exposure of the catalytically active *(100)* plane in the (VO)₂P₂O₇ catalyst in comparison to the conventional preparation methods.
4. The present process does not require hydrothermal reaction conditions for the preparation of (VO)₂P₂O₇ catalyst with selective exposure of the catalytically important *(100)* plane.
5. The present process does not require costly surfactant and mono dispersed colloidal templating agents during the preparation of (VO)₂P₂O₇ catalyst with selectively exposed *(100)* plane.

## Claims

1. A process for the preparation of vanadyl pyrophosphate catalyst with improved structural characteristics and useful for the selective oxidation of butane to maleic anhydride, which comprises:
(i) reducing V₂O₅ solid by an aqueous solution of NH₂OH.HCl in the presence of H₃PO₄ to obtain a blue solution w,
(ii) evaporating the solution to obtain a pasty mass,
(iii) aging the pasty mass to obtain a blue solid,
(iv) washing the solid with boiling water to remove the water soluble phases,
(v) drying the solid to form VOHPO₄.0.5H₂O phase,
(vi) grinding the dry VOHPO₄.0.5H₂O phase to a fine powder,
(vii) dispersing the VOHPO₄.0.5H₂O powder into a mixture of dimethyl formamide (DMF) and water (H₂O) and stirring the resulting slurry,
(viii) recovering the dispersed VOHPO₄.0.5H₂O powder from the slurry and washing with hot water,
(ix) drying the powder to obtain a VOHPO₄.0.5H₂O solid with selectively exposed *(001)* plane,
(x) calcining the VOHPO₄.0.5H₂O solid with selectively exposed *(001)* plane to obtain the desired (VO)₂P₂O₇ phase with selectively exposed *(100)* plane.

2. A process as claimed in claim 1 wherein the V₂O₅ solid is reduced in step (i) at a temperature in the range of 70-100°C while maintaining a P:V ratio in the reaction mixture in the range of 1:0.8 to 1:1.4.

3. A process as claimed in claim 1 wherein the pasty mass obtained in step (ii) is aged in step (iii) by heating to a temperature in the range of 100-150°C in air atmosphere for a period in the range of 12-36 hours.

4. A process as claimed in claim 1 wherein the washed solid obtained in step (iv) is dried in step (v) at a temperature in the range of 100-150°C for 6-16 hours in air atmosphere to form VOHPO₄.0.5H₂O phase.

5. A process as claimed in claim 1 wherein the dry VOHPO₄.0.5H₂O powder obtained in step (vi) is dispersed in step (vii) in a mixture of dimethyl formamide (DMF) and water (H₂O) wherein the DMF to H₂O ratio is in the range of 20:1-1:20 (v/v) and the resulting slurry is stirred for 1-6 hours at a temperature in the range of 50-100°C.

6. A process as claimed in claim 1 wherein the dispersed VOHPO₄.0.5H₂O powder is recovered from the slurry of step (vii) by filtration.

7. A process as claimed in claim 1 wherein the VOHPO₄.0.5H₂O solid with selectively exposed *(001)* plane obtained at the end of step (ix) is calcined in step (x) at a temperature in the range of 400-500°C under flowing nitrogen, at flow rate of 6-10 litre/hour for 1-6 hours to obtain (VO)₂P₂O₇ phase with selectively exposed *(100)* plane.

8. A process as claimed in claim 1 wherein the ratio of DMF to H₂O used in the mixture of DMF and H₂O is in the range of 2:1-1:2 (v/v).

9. A process as claimed in claim 1 wherein the temperature during stirring of VOHPO₄.0.5H₂O powder in DMF-H₂O mixture is in the range of 75-90°C.

10. A process as claimed in claim 1 wherein the selective exposure of the *(001)* plane in VOHPO₄.0.5H₂O is varied by changing the duration of DMF-H₂O treatment.

11. A process as claimed in claim 1 wherein the temperature used in calcination of VOHPO₄.0.5H₂O solid is in the range of 450-470°C with a gradual increase of temperature at a rate of 1-2°C/ minute under flowing nitrogen.

## Patentansprüche

1. Verfahren zur Herstellung von Vanadylpyrophosphatkatalysator mit verbesserten strukturellen Eigenschaften, der für die selektive Oxidation von Butan zu Maleinsäureanhydrid nützlich ist, das Folgendes umfasst:
(i) das Reduzieren von V₂O₅-Feststoff durch eine wässrige Lösung von NH₂OH.HCl in Gegenwart von H₃PO₄, um eine blaue Lösung w zu erhalten,
(ii) das Verdampfen der Lösung, um eine pastenförmige Masse zu erhalten,
(iii) das Altern der pastenförmigen Masse, um einen blauen Feststoff zu erhalten,
(iv) das Waschen des Feststoffs mit siedendem Wasser, um die wasserlöslichen Phasen zu entfernen,
(v) das Trocknen des Feststoffs, um VOHPO₄.0,5H₂O-Phase zu bilden,
(vi) das Mahlen der trockenen VOHPO₄.0,5H₂O-Phase zu einem feinen Pulver,
(vii) das Dispergieren des VOHPO₄.0,5H₂O-Pulvers in einer Mischung von Dimethylformamid (DMF) und Wasser (H₂O) und das Rühren der so gebildeten Aufschlämmung,
(viii) das Gewinnen des dispergierten VOHPO₄.0,5H₂O-Pulvers aus der Aufschlämmung und das Waschen mit heißem Wasser,
(ix) das Trocknen des Pulvers, um einen VOHPO₄.0,5H₂O-Feststoff mit selektiv exponierter (001)-Ebene zu erhalten,
(x) das Calcinieren des VOHPO₄.0,5H₂O-Feststoffs mit selektiv exponierterter (001)-Ebene, um die erwünsche (VO)₂P₂O₇-Phase mit selektiv exponierter (100)-Ebene zu erhalten.

2. Verfahren nach Anspruch 1, wobei der V₂O₅-Feststoff in Schritt (i) bei einer Temperatur im Bereich von 70 - 100°C reduziert wird, während ein P:V-Verhältnis in der Reaktionsmischung im Bereich von 1:0,8 bis 1:1,4 aufrechterhalten wird.

3. Verfahren nach Anspruch 1, wobei die pastenförmige Masse, die in Schritt (ii) erhalten wird, in Schritt (iii) durch Erhitzen auf eine Temperatur im Bereich von 100 - 150 °C in einer Luftatmosphäre für eine Zeitspanne im Bereich von 12 - 36 Stunden gealtert wird.

4. Verfahren nach Anspruch 1, wobei der gewaschene Feststoff, der in Schritt (iv) erhalten wird, in Schritt (v) bei einer Temperatur im Bereich von 100 - 150 °C 6 - 16 Stunden lang in einer Luftatmosphäre getrocknet wird, um die VOHPO₄.0,5H₂O-Phase zu bilden.

5. Verfahren nach Anspruch 1, wobei das in Schritt (vi) erhaltene trockene VOHPO₄.0,5H₂O-Pulver in Schritt (vii) in einer Mischung von Dimethylformamid (DMF) und Wasser (H₂O) dispergiert wird, wobei das Verhältnis von DMF zu H₂O im Bereich von 20:1 - 1:20 (Vol./Vol.) liegt und die so gebildete Aufschlämmung 1 - 6 Stunden lang bei einer Temperatur im Bereich von 50 - 100 °C gerührt wird.

6. Verfahren nach Anspruch 1, wobei das dispergierte VOHPO₄.0,5H₂O-Pulver aus der Aufschlämmung aus Schritt (vii) durch Filtrieren gewonnen wird.

7. Verfahren nach Anspruch 1, wobei der VOHPO₄.0,5H₂O-Feststoff mit selektiv exponierter (001)-Ebene, der am Ende von Schritt (ix) erhalten wird, in Schritt (x) bei einer Temperatur im Bereich von 400 - 500 °C unter fließendem Stickstoff mit einer Fließrate von 6 - 10 Litern/Stunde 1 - 6 Stunden lang calciniert wird, um die (VO)₂P₂O₇-Phase mit selektiv exponierter (100)-Ebene zu erhalten.

8. Verfahren nach Anspruch 1, wobei das Verhältnis von DMF zu H₂O, das in der Mischung von DMF und H₂O angewendet wird, im Bereich von 2:1 - 1:2 (Vol./Vol.) liegt.

9. Verfahren nach Anspruch 1, wobei die Temperatur während des Rührens von VOHPO₄.0,5H₂O-Pulver in der DMF-H₂O-Mischung im Bereich von 75 - 90°C liegt.

10. Verfahren nach Anspruch 1, wobei das selektive Exponieren der (001)-Ebene in VOHPO₄.0,5H₂O durch Ändern der Dauer der DMF-H₂O-Behandlung variiert wird.

11. Verfahren nach Anspruch 1, wobei die Temperatur, die beim Calcinieren von VOHPO₄.0,5H₂O-Feststoff angewendet wird, im Bereich von 450 - 470°C bei einer allmählichen Temperaturerhöhung mit einer Rate von 1 - 2°C/Minute unter fließendem Stickstoff liegt.

## Revendications

1. Procédé de préparation de catalyseur de pyrophosphate de vanadyle ayant des caractéristiques structurelles améliorées et utile pour l'oxydation sélective du butane en anhydride maléique, qui comprend :
(i) la réduction du solide de V₂O₅ par une solution aqueuse de NH₂OH.HCl en présence de H₃PO₄ pour obtenir une solution bleue w,
(ii) l'évaporation de la solution pour obtenir une masse pâteuse,
(iii) le vieillissement de la masse pâteuse pour obtenir un solide bleu,
(iv) le lavage du solide avec de l'eau bouillante pour éliminer les phases hydrosolubles,
(v) le séchage du solide pour former une phase VOHP0₄.0,5H₂O,
(vi) le broyage de la phase VOHPO₄.O,5H₂O sèche en une poudre fine,
(vii) la dispersion de la poudre de VOHPO₄.O,5H₂O dans un mélange de diméthylformamide (DMF) et d'eau (H₂O) et l'agitation de la suspension résultante,
(viii) la récupération de la poudre de VOHPO₄.0,5H₂O dispersée à partir de la suspension et le lavage avec de l'eau chaude,
(ix) le séchage de la poudre pour obtenir un solide de VOHPO₄.0,5H₂O avec un plan sélectivement exposé *(001),*
(x) la calcination du solide de VOHPO₄.0,5H₂O avec le plan sélectivement exposé *(001)* pour obtenir la phase (VO)₂P₂O₇ souhaitée avec le plan sélectivement exposé *(100).*

2. Procédé tel que revendiqué dans la revendication 1, dans lequel le solide de V₂O₅ est réduit dans l'étape (i) à une température dans la gamme de 70 à 100°C tout en maintenant un rapport P:V dans le mélange réactionnel dans la gamme de 1:0,8 à 1:1,4.

3. Procédé tel que revendiqué dans la revendication 1, dans lequel la masse pâteuse obtenue dans l'étape (ii) est vieillie dans l'étape (iii) par chauffage à une température dans la gamme de 100 à 150°C dans l'atmosphère ambiante pendant une période dans la gamme de 12 à 36 heures.

4. Procédé tel que revendiqué dans la revendication 1, dans lequel le solide lavé obtenu dans l'étape (iv) est séché dans l'étape (v) à une température dans la gamme de 100 à 150 °C pendant 6 à 16 heures dans l'atmosphère ambiante pour former la phase VOHPO₄.0,5H₂O.

5. Procédé tel que revendiqué dans la revendication 1, dans lequel la poudre de VOHP0₄.O,5H₂O sèche obtenue dans l'étape (vi) est dispersée dans l'étape (vii) dans un mélange de diméthylformamide (DMF) et d'eau (H₂O) dans lequel le rapport du DMF à l'eau est dans la gamme de 20:1 à 1:20 (v/v) et la suspension résultante est agitée pendant 1 à 16 heures à une température dans la gamme de 50 à 100°C.

6. Procédé tel que revendiqué dans la revendication 1, dans lequel la poudre de VOHPO₄.0,5H₂O dispersée est récupérée à partir de la suspension de l'étape (vii) par filtration.

7. Procédé tel que revendiqué dans la revendication 1, dans lequel le solide de VOHPO₄.0,5H₂O avec le plan sélectivement exposé *(001)* obtenu à la fin de l'étape (ix) est calciné dans l'étape (x) à une température dans la gamme de 400 à 500°C sous écoulement d'azote, à un débit de 6 à 10 litres/heure pendant 1 à 6 heures pour obtenir la phase (VO)₂P₂O₇ avec le plan sélectivement exposé *(100).*

8. Procédé tel que revendiqué dans la revendication 1, dans lequel le rapport du DMF à l'H₂O utilisé dans le mélange de DMF et d'H₂O est dans la gamme de 2:1 à 1:2 (v/v).

9. Procédé tel que revendiqué dans la revendication 1, dans lequel la température pendant l'agitation de la poudre de VOHPO₄.0,5H₂O dans le mélange DMF-H₂O est dans la gamme de 75 à 90°C.

10. Procédé tel que revendiqué dans la revendication 1, dans lequel l'exposition sélective du plan *(001)* dans VOHPO₄.0,5H₂O est variée en modifiant la durée du traitement au DMF-H₂O.

11. Procédé tel que revendiqué dans la revendication 1, dans lequel la température utilisée lors de la calcination du solide de VOHPO₄.0,5H₂O est dans la gamme de 450 à 470°C avec une augmentation graduelle de la température à une vitesse de 1 à 2°C/minute sous écoulement d'azote.
